# EUROPEAN PATENT APPLICATION

(11) **EP 3 973 897 A1**
(43) Date of publication of application: **30.03.2022**
(21) Application number: 21197906.7
(22) Date of filing: 21.09.2021
(51) Int. Cl.: A61B 17/34

(54) **SURGICAL ACCESS DEVICE WITH CHECK VALVE AND PLURAL DISCS**

(30) Priority: 23.09.2020 US 202017030186
(71) Applicant: Covidien LP, Mansfield, MA 02048 (US)
(72) Inventor: EBERSOLE, Garrett P., North Haven, CT Connecticut 06473 (US); BARIL, Jacob C., North Haven, CT Connecticut 06473 (US); DININO, Matthew A., North Haven, CT Connecticut 06473 (US); THOMAS, Justin, North Haven, CT Connecticut 06473 (US); BANERJEE, Saumya, North Haven, CT Connecticut 06473 (US); PILLETERE, Roy J, North haven, CT Connecticut 06473 (US); LaPIERRE, Nicolette R., North Haven, CT Connecticut 06473 (US)
(74) Representative: Maschio, Antonio

(57) **Abstract**

A surgical access device includes a check valve having baffling discs, a tube, and a housing. The housing has first and second housing halves. Baffling discs are configured to form a lumen. Each baffling disc is a conical annulus and is stacked adjacent to another baffling disc and spaced apart via spacers disposed on each baffling disc. The spacers help define a first gap between adjacent baffling discs for defining a first fluid path. A cover is coupled to the housing and permits access to the surgical access device. The housing is configured to receive the tube and the baffling discs, and the baffling discs are spaced apart from the first and second housing halves defining a second gap therebetween for defining a second fluid path. The first and second gaps form fluid convection channels for guiding fluid flowing in a proximal direction back into itself.

## Description

### FIELD

The present disclosure is generally related to tunneling through body tissue and accessing a surgical site. More particularly, this disclosure relates to a surgical access device with a check valve having plural discs.

### BACKGROUND

Minimally invasive surgery is enabled by the creation of working space, i.e., a pneumoperitoneum, inside the abdominal cavity. The pneumoperitoneum is created by the introduction of an insufflation fluid, generally carbon dioxide, and maintained by a system of seals inside access devices placed through the abdominal wall. Surgical access devices, such as trocars, cannulas, obturator tube assemblies, and the like, generally have a seal located at a proximal end, which allows instruments to pass through while inhibiting fluids from escaping from the abdominal cavity. Various types of valves are available, such as spring-loaded, magnetic trap door, and trumpet with each type of valve offering different characteristics in terms of leakage, mode of operation, and location on the cannula.

Cannulas often contain a duckbill valve to seal off the pneumoperitoneum when no instrument is present and an instrument seal to seal against an inserted instrument. Both seals may interact with an inserted surgical instrument. This interaction is perceived by a surgeon as friction. Additionally, these interactions increase the chance for the generation of particulate matter as instruments dilate and pass through the seals.

Seals are also used in a variety of medical applications, such as those used in vascular access. Often, these seals may still result in blood loss, in the vascular access case, or other fluid loss such as a loss of insufflation fluid. A seal capable of inhibiting the escape of matter while minimizing interaction with surgical or medical instruments inserted through the seal is desired.

### SUMMARY

This disclosure generally relates to a surgical access device including a tube and a housing. The housing includes a first housing half and a second housing half. Baffling discs are configured in the housing to form a lumen and each baffling disc is a conical annulus. Further, each baffling disc is stacked adjacent to another baffling disc. The adjacent baffling disc is spaced apart via spacers disposed on each baffling disc so as to define a first gap between adjacent baffling discs that define a first fluid path. A cover is coupled to a proximal end of the housing and configured to control access to the lumen. The first and second housing halves of the housing are configured to receive the tube and the baffling discs. The baffling discs are spaced apart from the first and second housing halves that define a second gap therebetween that defines a second fluid path. The first and second gaps form fluid convection channels for guiding fluid flowing in a proximal direction.

In aspects, the first and second housing halves may include recesses configured to follow an upper guide of each baffling disc so as to further define the second gap therebetween. When the baffling discs are received by the first and second housing halves, fluid flowing in the proximal direction is encouraged to flow through the fluid convection channels, through the first gap between each baffling disc, through the second gap between the baffling discs and the recesses, and back around against the fluid flowing in the proximal direction so as to reduce net flow in the proximal direction.

In other aspects, each baffling disc may have a cross-sectional profile of an airfoil, with an upper guide and a lower guide, wherein the upper guide has a radius that is larger than the radius of the lower guide.

In yet another aspect, the lumen formed by the baffling discs may be configured to receive a laparoscopic instrument.

In particular aspects, the surgical access device may include an accessory attachment configured to couple to the cover.

In another aspect, the accessory attachment may include at least one luer fitting.

In aspects, the housing may include five baffling discs.

In additional aspects, the baffling discs may define a lumen that is as long as at least a quarter of a length of the tube.

In yet other aspects, the tube may have a flared proximal end and the two housing halves may have distal portions configured to couple to and engage the flared proximal end.

In other aspects, the lumen formed by the baffling discs may have a diameter equal to an inner diameter of the tube and is concentric and axially aligned with the tube.

In accordance with another aspect of this disclosure, a surgical assembly includes a surgical instrument having a shaft and a surgical access assembly. The surgical access assembly includes a housing having open proximal and distal ends, a tube extending from the housing, and baffling discs disposed within the housing. Each baffling disc has a cross-sectional profile of an airfoil and is spaced apart from an adjacent baffling disc thereby defining a first gap therebetween. Each baffling disc spaced from an inner surface of the housing thereby defining a second gap therebetween. The first and second gaps form fluid convection channel for guiding fluid flowing in the housing.

In aspects, the housing may include first and second housing halves.

In a further aspect, each housing half may include recesses configured to follow an upper guide of each baffling disc so as to further define the second gap therebetween and fluid flowing in a proximal direction is encouraged to flow through the fluid convection channels, through the first gap between each baffling disc, through the second gap between the baffling discs and the recesses, and back around against the fluid flowing in the proximal direction so as to reduce net flow in the proximal direction.

In an aspect, the cross-sectional profile of an airfoil may be defined by an upper guide and a lower guide, wherein the upper guide has a radius that is larger than a radius of the lower guide.

In another aspect, the tube may have a flared proximal end and the first and second housing halves may have distal portions configured to couple to and engage the flared proximal end.

In further aspects, each baffling disc may be a conical annulus.

In yet even further aspects, a narrow portion of the conical annulus of each baffling disc may be positioned distally relative to a user, and is positioned adjacent a wider portion of the conical annulus of an adjacent baffling disc.

In an aspect, a proximalmost portion of the tube may be spaced apart from the distalmost baffling disc and is configured to follow the narrow portion of the conical annulus of the distalmost baffling disc so as to define a third gap therebetween.

In yet other aspects, a cover may be disposed on a proximalmost portion of the housing of the surgical access assembly.

In further aspects, the cover may be spaced apart from the proximalmost baffling disc and is configured to follow a portion of the proximalmost baffling disc so as to define a third gap therebetween that forms an extension of the proximalmost fluid convection channel.

The details of one or more aspects of the disclosure are set forth in the accompanying drawings and the description below. Other features, objects, and advantages of the techniques described in this disclosure will be apparent from the description and drawings, and from the claims.

### DESCRIPTION OF DRAWINGS

The accompanying drawings, which are incorporated in and constitute a part of this specification, illustrate aspects and features of the disclosure and, together with the detailed description below, serve to further explain the disclosure, in which:
FIG. 1 is a perspective view of a surgical access device including a housing with a check valve;
FIG. 2 is an exploded perspective view, with parts separated, of the surgical access device shown in FIG. 1;
FIG. 3 is a detailed view of a housing half of the housing of the surgical access device shown in FIGS. 1 and 2;
FIG. 4 is a top perspective view of a baffling disc;
FIG. 5 is a bottom perspective view of the baffling disc of FIG. 4;
FIG. 6 is a cross-sectional view of the baffling disc of FIG. 4 taken along section line 6-6;
FIG. 7 is a cross-sectional view of the housing half of FIG. 1 taken along section line 7-7;
FIG. 8 is a cross-sectional view of the housing half of FIG. 1 taken along section line 8-8;
FIG. 9 is a functional view of the surgical access device of FIG. 1 inserted through an opening in tissue with an accessory attachment coupled to the housing and a surgical instrument inserted therein;
FIG. 10 is a cross-sectional view of the housing and accessory attachment of FIG. 9 taken along section line 10-10; and
FIG. 11 is an enlarged view of the area of detail of FIG. 10 illustrating a flow path around the baffling discs.

Further details and various aspects of this disclosure are described in more detail below with reference to the appended figures.

### DETAILED DESCRIPTION

Aspects of the presently disclosed surgical access devices are described in detail with reference to the drawings, in which like reference numerals designate identical or corresponding elements in each of the several views. However, it is to be understood that the disclosed devices are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

Descriptions of technical features of an illustrative surgical access device having a check valve with plural baffling discs in accordance with the disclosure should typically be considered as available and applicable to other similar features of another device of the disclosure. Accordingly, technical features described herein in connection with one illustrative surgical access device may apply to other devices of the disclosure, and thus duplicative descriptions may be omitted herein.

As used herein the term "distal" refers to that portion of the surgical access device, or component thereof, farther from the user, while the term "proximal" refers to that portion of the surgical access device, or component thereof, closer to the user.

This disclosure relates to a surgical access device with a check valve formed from a plurality of baffling discs. The baffling discs provide little to no fluid resistance in one direction, but in the opposite direction, they passively (i.e., without any moving parts) and palpably impede fluid flow. In a typical trocar or cannula, fluids or gasses in or introduced into the housing tend to push their way through the tube of the trocar or cannula. Accordingly, many devices employ instrument seals, zero seals, duckbill seals, and the like to inhibit fluid or gasses from escaping out the surgical access device. The check valve of this disclosure inhibits fluid or gasses from escaping out the surgical access device and does not interfere with a surgical instrument inserted therein. Baffling discs direct fluid flowing in a distal to proximal direction (exiting direction) back into and against fluid flow in the exiting direction, creating eddies, surges, and/or reversed flow to impede flow in the exiting direction. In other terms, the baffling discs turn the flow back in on itself, slowing and inhibiting its motion in the exiting direction. The baffling discs provide a seal or check valve that does not interfere with any instruments inserted therethrough and provide an unobstructed path through the surgical access device.

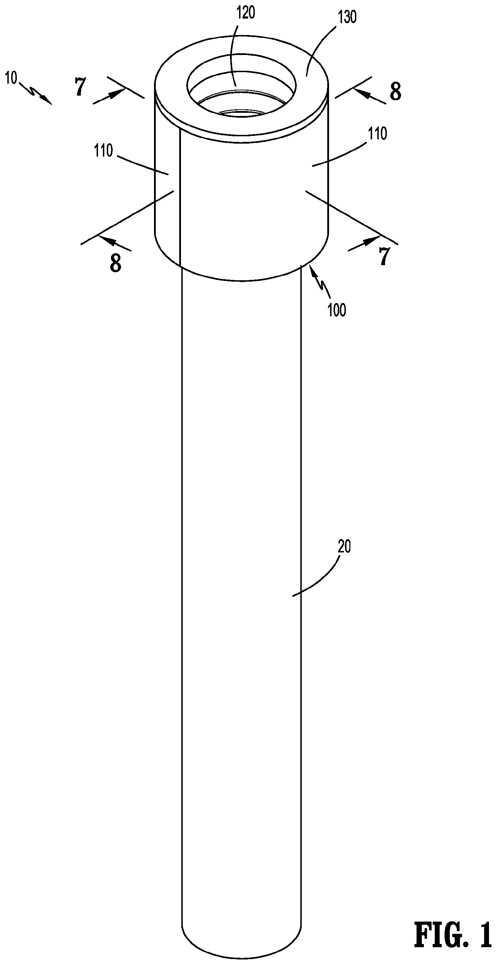

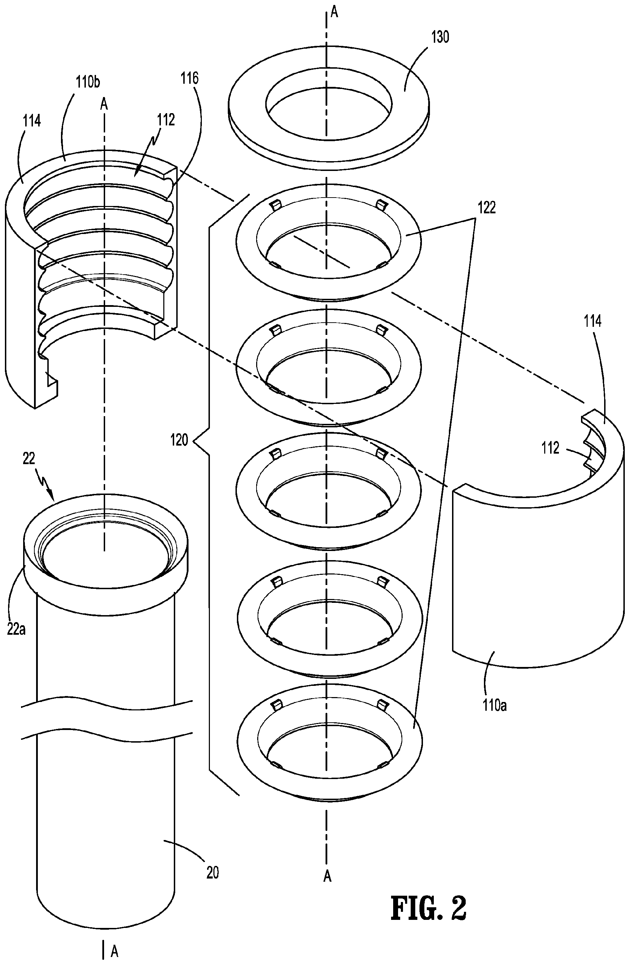

Referring to FIGS. 1 and 2, a surgical access device 10 of this disclosure is illustrated and generally includes a tube or cannula 20, a housing 100, a check valve 120 received in the housing 100, and a cover 130. A plurality of baffling discs 122 forms the check valve 120 to readily permit flow in one direction (i.e., distally) and restrict flow in the opposite direction (i.e., proximally). The housing 100 is positioned at a proximal portion of the tube or cannula 20. The terms "tube," "elongated tubular member," and "cannula" are used interchangeably herein and are intended to include any or all of the mechanisms known in the art for separating tissue planes in a surgical procedure and/or for creating access through which a surgical instrument may be inserted (such as an obturator, grasper, endoscopic camera, electric cautery tool, etc.) The housing 100 includes a first housing half 110a and a second housing half 110b, the first and second housing halves 110a, 110b are configured to receive the plurality of baffling discs 122 that form the check valve 120. The inner facing walls 112 of each housing half 110a, 110b are mirror images of one another. The housing 100 has a cover 130 to couple to and secure the housing halves 110a, 110b together. Additionally, the cover 130 is configured to seal off the top faces 114 of the housing halves 110a, 110b and crevice that is left when the two housing halves 110a, 110b are in contact. The housing halves 110a, 110b are joined using techniques known in the art such as adhesives, ultrasonic welding, etc.

The housing 100, the tube 20, the cover 130, and the plurality of baffling discs 122 may be made from a variety of biocompatible materials including metals, aluminum, stainless steel, plastic, polymers, or combinations thereof. Any material suitable for use in a cannula or trocar known to those of ordinary skill in the art may be used to form any one or all components of the surgical access device 10. In aspects, the various components may be manufactured using 3D printing.

An exploded view of the surgical access device 10 is shown in FIG. 2 and illustrates an example configuration for assembling the housing 100 to form the check valve 120. The baffling discs 122 of the check valve 120 are stacked and adjacent to one another. Each housing half 110a, 110b has a section to receive a corresponding baffling disc 122, such that when the housing halves 110a, 110b are brought together they form a housing 100 around the baffling discs 122. The check valve 120 may include any suitable number of baffling discs 122, such as five (5) baffling discs 122 as shown in FIG. 2. In aspects, a sufficient number of baffling discs 122 may be used such that the check valve 120 and housing 100 form the tube 20. In other aspects, the check valve 120 may have a sufficient number of baffling discs 122 such that the check valve 120 has a length equal to half a length of the tube 20.

Each baffling disc 122 may have identical geometry as shown in FIGS. 2, and 4-6, but it is contemplated that the proximalmost baffling disc 122 or the distalmost baffling disc 122 may have a different geometry. It is further contemplated that each baffling disc 122 may have a different geometry.

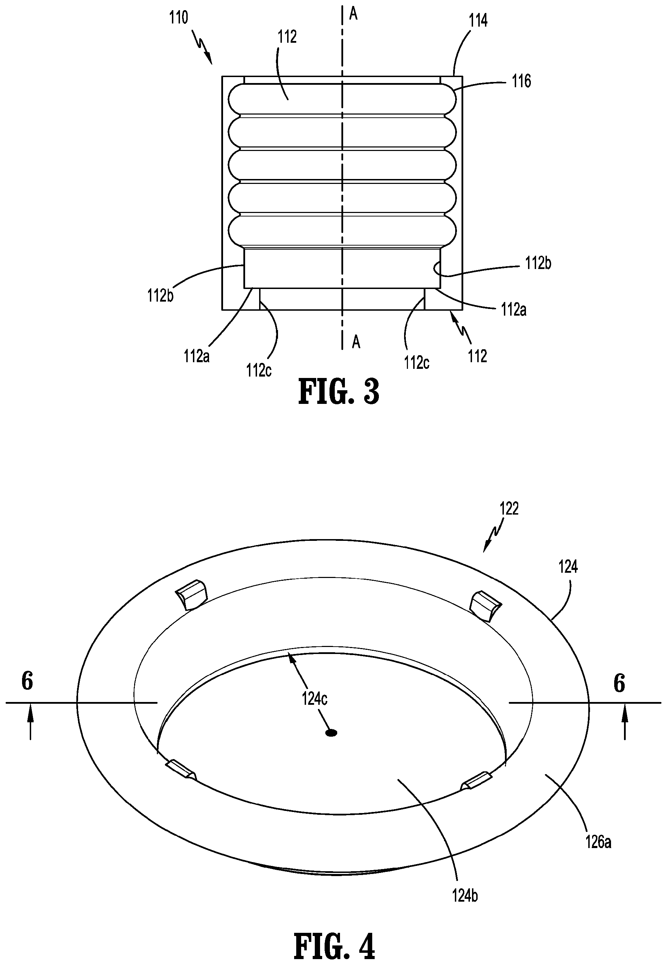

With additional reference to FIG. 3, a front view of a housing half 110 is illustrated and configured to receive five baffling discs 122 and the flared proximal end 22 of the tube 20. The housing 100 is also configured to receive a proximal portion of the tube 20. The tube 20 has a flared proximal end 22 (i.e., a flange) configured to couple with and be retained between the housing halves 110a, 110b of the housing 100. The terms "flared proximal end" and "flange" are used to refer to the same proximal portion of a tube configured to couple to a housing and are used interchangeably throughout this disclosure. The inner facing walls 112 of the housing 100 may have contours 112b at a distal portion thereof configured to receive the flared proximal end 22. In aspects, the distal portion of the housing 100 includes a shelf 112a for retaining the flared proximal end or flange 22 of the tube 20. In aspects, the flange 22 may be coupled to the shelf 112a by a friction fit between the housing halves 110a, 110b or ultrasonically welded together, thereby retaining the tube 20 and the housing 100 in position. In aspects, the housing 100 may couple to the cannula 20 by any means known by those of ordinary skill in the art. For example, the flared proximal end 22 may have external or internal threads for screwing into and interlocking with external or internal threads at a distal portion of the housing 100. The housing 100 and tube 20 when coupled together form the surgical access device 10 and create a shaft with a common longitudinal axis A-A.

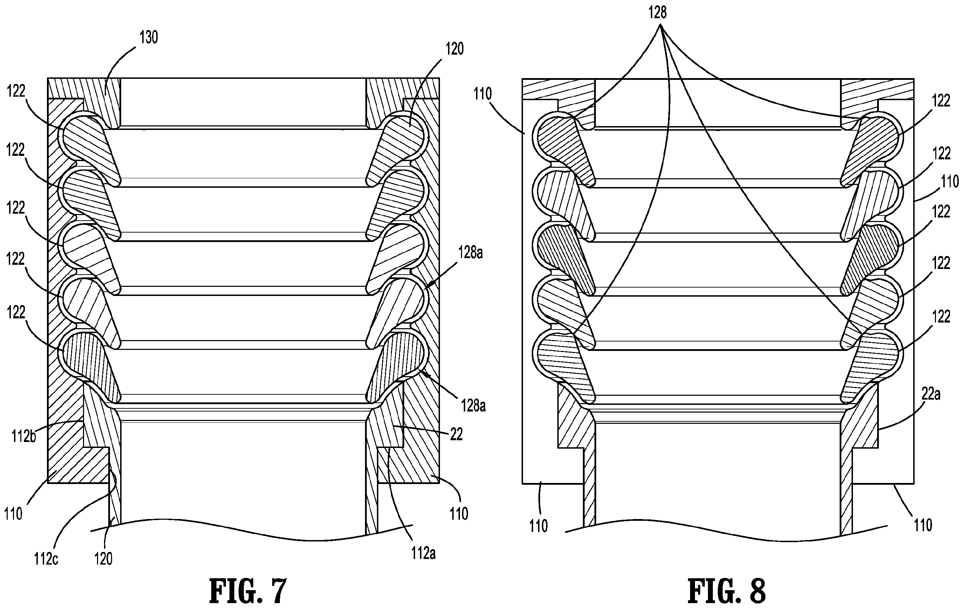

The housing halves 110a, 110b when coupled together define a lumen through the housing 100. Typically, the lumen is cylindrical and forms an extension of the shaft of the tube 20. The lumen has a radius larger than the largest radius of a baffling disc 122 and/ or the radius of the tube 20. In aspects, at a distal portion of the housing halves 110a, 110b, the lumen has a radius no larger than the radius of the tube or cannula 20. Recesses 116 are cut into the inner facing wall of each housing half 110a, 110b and are configured to receive a portion of a baffling disc 122. The recesses 116 may be semi-cylindrical recesses having a radius of curvature larger than the radius of curvature of a baffling disc 122. The inner facing walls 112 extend distally past the distalmost semi-cylindrical recess 116, and at a distal portion have a smooth, planar wall or contours 112b, configured to engage the outer wall of the flared proximal end 22 of the tube 20. Engagement arms 112c protrude from the distal portion of each housing half 110a, 110b, and define a shelf that is configured to couple below the flared proximal end 22 (see FIGS. 7-8). The bottom surface of the flared proximal end 22 of the tube 20 engages the top surfaces of the engagement arms 112c (see FIG. 7). The inner facing walls 112 similarly engage the outer walls 22a of the flared proximal end 22 of the tube 20. When the two housing halves 110a, 110b are in contact, a fluid-tight seal is formed therebetween and the flared proximal end 22 of the tube 20 (see FIGS. 7 and 8). The two housing halves 110a, 110b may each have male connectors and/or female receptacles (not shown) for facilitating a snap-fit engagement and contact between one another. The two housing halves 110a, 110b may be glued or ultrasonically welded.

Turning now to FIGS. 4-6, various aspects and details of an exemplary baffling disc 122 are shown. Each baffling disc 122 is a conical annulus 124 defining an aperture 124b, where the aperture 124b is defined by an inner radius 124c of the baffling disc 122 that is approximately the same radius as the tube 20 (see FIG. 7). Each baffling disc 122 has upper and lower surfaces 126a, 126b, and the upper and lower surfaces 126a, 126b have contours that are either convex, concave, flat, or a combination thereof. The baffling discs 122 are configured such that when stacked adjacent to one another, the aperture 124b of each baffling disc 122 aligns with the aperture 124b of an adjacent baffling disc 122 to further define the lumen of the housing 100 and the narrowed ends of the conical annuli 124 are distally positioned relative to the wider ends.

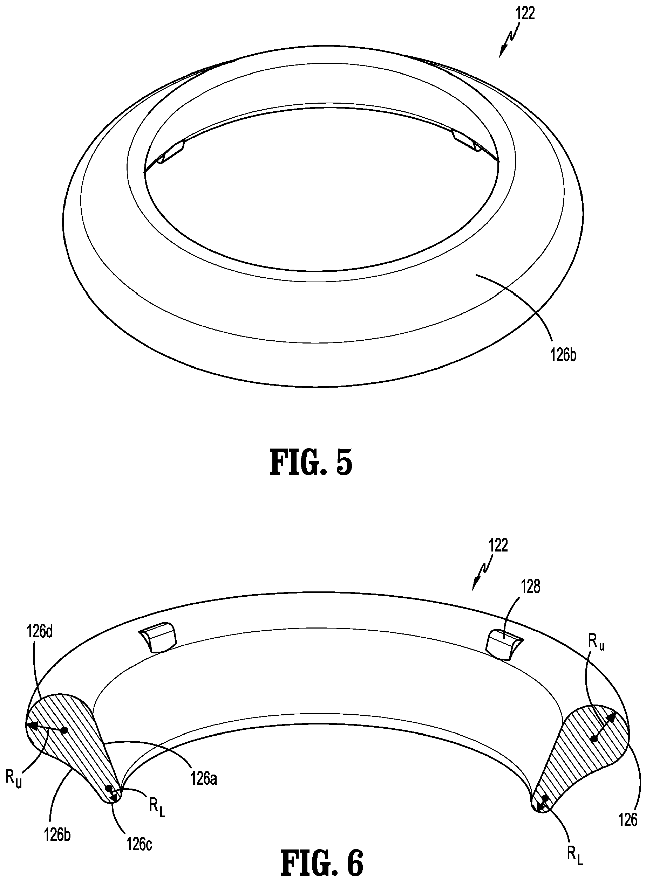

In aspects, the cross-sectional profile 126 of each baffling disc resembles an airfoil as shown in FIG. 6. The airfoil cross-sectional profile 126 is configured to guide fluid to flow from a lower guide 126c around the upper surface 126a, bend around an upper guide 126d, and flow back down the lower surface 126b. In other aspects, the fluid may be guided first past the lower surface, around the upper guide 126d and back down the upper surface 126a. The lower guide 126c has a lower guide radius "R_{L}" and the upper guide 126d has an upper guide radius "Rᵤ". In aspects, the lower guide radius "R_{L}" is smaller than the upper guide radius "Rᵤ". The upper guide radius "Rᵤ" may be at least twice the radius of the lower guide radius "R_{L}". In aspects, the upper surface 126a is approximately flat from the upper guide 126d to the lower guide 126c. The lower surface 126b may be convex from the lower guide 126c to about half the distance to the upper guide 126d and concave from about half the distance from the lower guide 126c until the upper guide 126d. When the baffling discs 122 are stacked, the lower guides 126c align vertically with each other and the inner wall of the tube 20 when coupled thereto.

The height of each baffling disc 122, as measured vertically from the proximalmost portion of the upper surface 126a to the distalmost portion of the lower surface 126b, may be from about 1 mm to about 20 mm. In aspects, the height is about 5 mm to about 15 mm. When stacked together to form the plurality of baffling discs 120, the height measured from the proximalmost portion of the proximalmost baffling disc 122 to the distalmost portion of the distalmost baffling disc 122 may be from about 10 mm to about 100 mm, and generally may be about 50 mm to about 70 mm.

The thickness of each baffling disc 122, as measured from the inner radius to an outer radius, or from the innermost portion to the outermost portion of each baffling disc, may be from about 5 mm to about 20 mm.

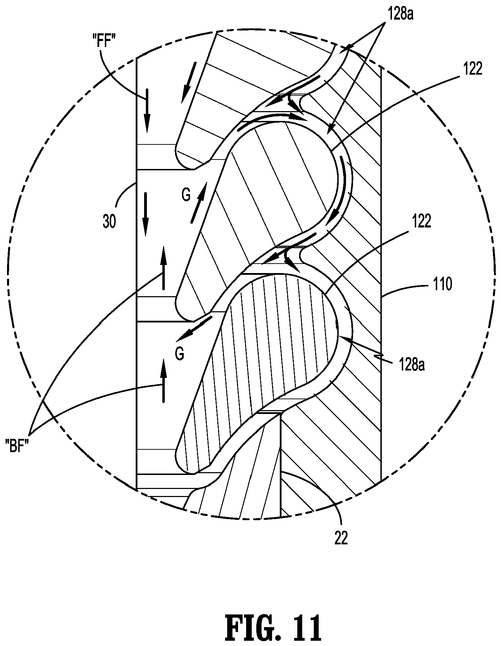

Each baffling disc 122 includes at least two (2) spacers 128. In aspects, each baffling disc 122 includes four (4) spacers 128. The spacers 128 are configured to prevent the upper surface 126a of a baffling disc 122 from coming into contact with the entirety of the lower surface 126b of a baffling disc 122 stacked on top of it. The spacers 128 are configured to create a gap between two baffling discs 122 such that a fluid may pass through the gap. The gaps formed by a plurality of stacked baffling discs 120 generate fluid convection channels 128a (see FIGS. 7 and 11) that passively pull or guide fluid through the gaps. The spacers 128 may be equally or randomly distributed along the upper surface 126a of each baffling disc. In aspects, the spacers 128 are positioned at a proximal portion of the upper surface 126a. In other aspects, the spacers 128 may be positioned on the lower surface. Spacers 128 may be any suitable shape, such as cones, polyhedrons, or prisms.

The spacers 128 may form directional fins (not shown) that guide the fluid to form a vortex. Alternatively, directional fins distinct from the spacers 128 may be disposed on the baffling discs 122. In yet other alternatives, directional channels grooved into the baffling discs 122 may direct the fluid to form a vortex. In other aspects, the directional fins may be disposed on the semi-cylindrical recesses.

The spacers 128 may be extended partially or completely in an arc around the upper surface 126a of each baffling disc 122. For example, if a baffling disc 122 has four spacers 128, two of the spacers 128 may be elongated and disposed on the upper surface 126a such that the distance between the two elongated spacers 128 is less than the distance between the other two spacers 128. In aspects, the spacers 128 may fully cover the upper surface 126a of a baffling disc, such that there is no gap exists between adjacent baffling discs 122. Baffling discs 122 may alternately include spacers 128 that cover various portions of the upper surface 126a to control fluid flow through the gaps between the baffling discs 122 and between the plurality of baffling discs 120 and the housing halves 110a, 110b. For example, every other baffling disc 122 of a plurality of baffling discs 120 may have spacers 128 covering a majority or all of the upper surface 126a of every other baffling disc 122. In another example, the spacers may cover 75% of the upper surface 126a, with the remaining uncovered portions of the upper surface 126a of each baffling disc are aligned. Spacers 128 of any size and shape may be used to partially or fully cover the upper surface 126a of a baffling disc 122 and may be arranged in any desired manner to produce a desirable effect on fluid flowing between, and about the baffling discs 122.

The baffling discs 122 may be formed from a single piece of material or formed via multiple pieces. The baffling discs 122 may be made via injection molding or 3D printing. It is contemplated that the plurality of discs may be formed as a single unit via 3D printing. In aspects, the baffling discs 122 may be made from a variety of materials such as stainless steel or medical grade plastics. The spacers 128 may be formed as a part of a baffling disc or may be attached as a separate piece to a baffling disc.

With reference to FIGS. 7 and 8, sectional views of the surgical access device 10 of FIG. 1 illustrate the housing 100, baffling discs 122, cover 130, and tube 20 coupled together. In aspects, the check valve 120 includes five (5) baffling discs 122 stacked together to define the lumen through the apertures 124b of the baffling discs 122. Any desirable quantity of baffling discs 122 may be used and received by the housing 100. The check valve 120 is disposed on a proximal portion of the tube 20, such that the distalmost baffling disc 122 is above all or a portion of the flared proximal end's 22 upper surface (see FIG. 7). The check valve 120 is approximately concentric with the tube 20. The upper surface of the flared proximal end or flange 22 is configured to receive a baffling disc 122 and may have similar contours to the lower surface 126b of the baffling discs 122. The flared proximal end 22 may have spacers 128 (not shown) so as to create a gap, and therefore a fluid convection channel 128a, between the distalmost baffling disc 122 and the flared proximal end 22. In aspects, the check valve 120 may be disposed on the flared proximal end 22 such that little to no gap exists.

The housing halves 110a, 110b receive the check valve 120 and the flared proximal end 22 therebetween, thereby sandwiching the check valve 120 and flared proximal end 22. The housing halves 110a, 110b define a fluid tight structure when coupled together. As previously discussed, the housing halves 110a, 110b have distal portions with engagement arms 112c for coupling below the flared proximal end 22 of the tube 20. As illustrated in FIGS. 7 and 8, the engagement arms 112c surround and are in contact with the flared proximal end 22 of the tube 20. The flange may rest on a shelf 112a. The check valve 120 and tube 20, once received by the housing 100, form a uniform cannula with a common longitudinal axis A-A.

The baffling discs 122 are stacked together to form a plurality of fluid convection channels 128a via the gaps between them (FIG. 7). As illustrated in FIG. 8, the spacers 128 allow the baffling discs 122 to maintain the gap between one another. At the spacers 128, the airfoil cross-sectional profile 126 has a hump indicating the spacer. The two housing halves 110a, 110b have semi-cylindrical recesses 116 that further define the fluid convection channels 128a. In aspects, the inner walls 112 of the two housing halves 110a, 110b are contoured to guide fluid flowing through the fluid convection channels 128a of the baffling discs 122 around the upper guides 126d of the baffling discs 122 back into the main flow stream around.

With continuing reference to FIGS. 7 and 8, a cover 130 is provided for covering the housing halves 110a, 110b of the housing 100, and the proximalmost baffling disc 122. It is generally a solid ring with an outer radius that is at least is as large as an outer radius of a housing half 110a, 110b of the housing 100. The inner radius is at least as large as the inner radius of a baffling disc 122. Thus, the cover 130 also defines an aperture with inner walls that are approximately aligned with the inner wall of the tube 20 and the lower guides of the baffling discs 122. When coupled to the housing 100, the cover 130 is concentric with the apertures of the baffling discs 122. The cover 130 also retains the proximalmost baffling disc 122 within the housing 110.

The bottom surface of the cover 130 may be contoured to follow the upper surface 126a of the proximalmost baffling disc 122 and the two housing halves 110a, 110b. The bottom surface of the cover 130 is distanced and spaced apart from the proximalmost baffling disc 122 (FIG. 7) but may be in contact with a spacer 128 disposed on the upper surface 126a of the proximalmost baffling disc (FIG. 8). In aspects, the proximalmost baffling disc 122 and cover 130 may be adjacent one another with no gap therebetween. In such an instance, the proximalmost baffling disc 122 may not include spacers 128 disposed on its upper surface 126a. The bottom surface of the cover 130 is contoured to be adjacent to and in contact with the two housing halves 110a, 110b of the housing 100. The cover 130 may be secured to the two housing halves 110a, 110b using glue, ultrasonic welding, or other well-known methods for securing parts of a cannula or trocar commonly used by those of ordinary skill in the art.

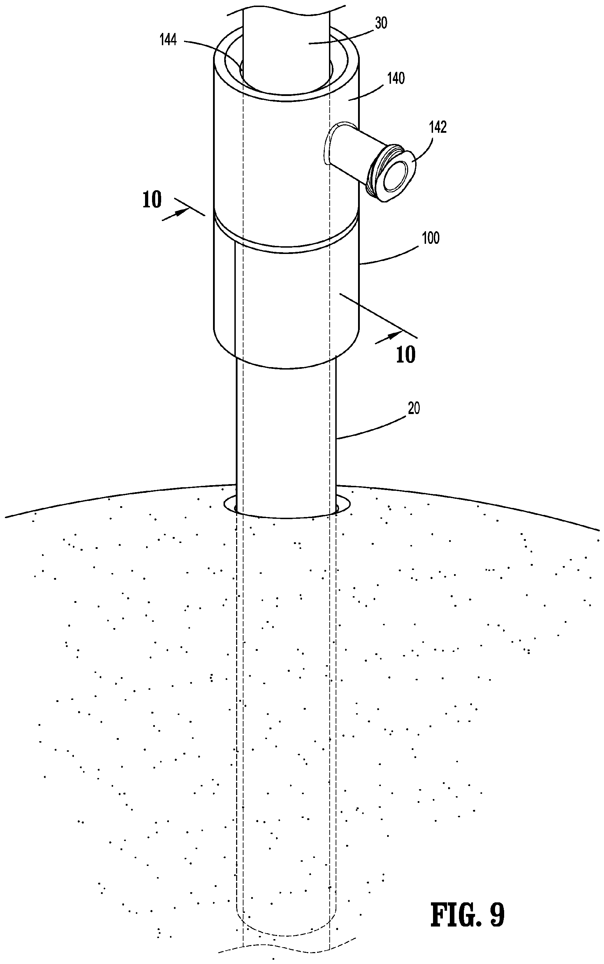

Referring now to FIG. 9, the surgical access device 10 may further include an accessory attachment 140 disposed on the housing 100. The accessory attachment 140 may be glued, ultrasonically welded, or otherwise coupled to the cover of the housing 100. The accessory attachment 140 has outer walls that are aligned with the housing 100 and has a passage 144 aligned and concentric with the lumen extending through the housing 100. The accessory attachment 140 includes a luer fitting 142 or port for supplying a fluid. The fluid may be insufflation fluid, carbon dioxide, saline solutions, or any fluid desired to be used by a surgeon or medical professional. In aspects, more than one luer fitting 142 or port may be included with the accessory attachment 140.

The accessory attachment 140 may be configured to provide easy surgical instrument introduction. As illustrated by FIG. 9, an instrument may be inserted through the surgical access device 10 to the working space within the pneumoperitoneum. A cap or plug (not shown) may be provided to seal the accessory attachment 140, and therefore the surgical access device 10, from the environment to prevent contamination or introduction of debris into the surgical access device 10 when an instrument is not inserted therein. In aspects, a plug may extend to the distalmost baffling disc 122 in order to force fluid into the convection channels 128a of the check valve 120.

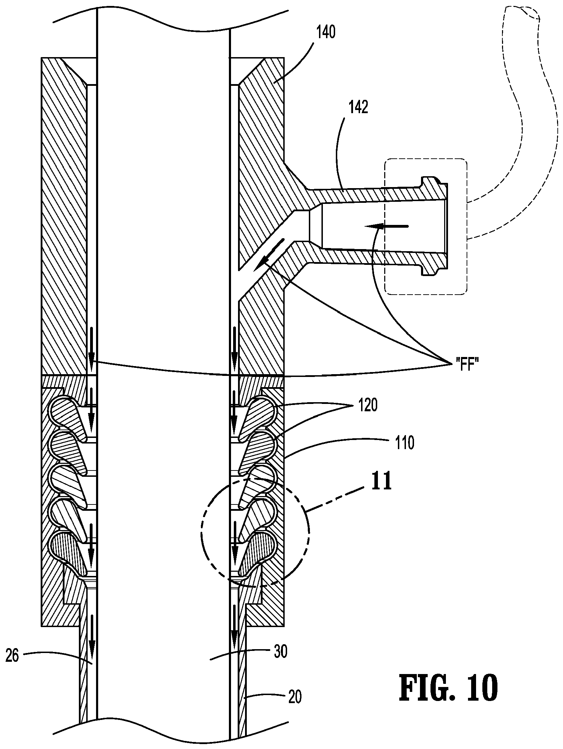

With reference to FIGS. 10 and 11, a sectional view of the surgical access device 10 of FIG. 9 with a surgical instrument 30 inserted therein is illustrated in FIG. 10, and FIG. 11 is a magnified section of FIG. 10 illustrating a fluid path through the convection channels 128a defined by the baffling discs 122. A surgical instrument 30, such as an endoscope, grasper, or the like, is inserted into the surgical access device 10. As shown in FIGS. 10 and 11, the check valve 120 does not interfere with a surgical instrument 30 inserted therein, allowing a surgeon to "feel" movement of the surgical instrument without any friction that typically may be felt using other seals.

A fluid supply is coupled to the luer fitting 142, and supplies a fluid marked by arrows "FF" (fluid flow) to the surgical site through the tube 20. The surgical instrument 30 may have a diameter that is slightly smaller than the inner diameter of the cannula. Fluid in the tube 20 is forced between the outer surface of the surgical instrument 30 and the inner surface of the tube 20 of the surgical access device 10. The lumen of the surgical access device 10 may have a variety of diameters depending on the surgical task to be performed, area of operation, preference of a surgeon, etc. to accommodate variously sized surgical instruments. Typically, an outer diameter of a surgical instrument inserted into the cannula is slightly smaller than the inner diameter (shaft diameter) of the cannula assembly. For example, the check valve 120, the tube 20, and the housing 100 may define a lumen having a 12mm diameter to accommodate instruments with an outside diameter less than 12mm. The difference in diameters defines a gap 26 for fluid flow between the inner surface of the tube 20 and the outer surface of the surgical instrument 30.

As shown in FIG. 10, fluid entering the surgical access device 10 via the luer fitting 142 is able to flow in the gap 26 between the tube 20 and the surgical instrument 30. Fluid is able to flow distally past the check valve 120 and the surgical instrument inserted 30 therein. However, fluid flow in the exiting (i.e., proximal) direction (back-flow) is impeded by the check valve 120, as illustrated in FIG. 11. The back-flow is pulled into the fluid convection channels 128a, guided around the upper surface 126a and upper guides of each baffling disc, and back down the lower surface. Some of the fluid flowing down the lower surface is caught by the fluid stream following the upper surface 126a of the baffling disc below as shown in FIG. 11. Eddies and swirls moving against the back-flow may force the fluid in the exiting direction to slow down. Insufflation fluid entering the shaft via the luer fitting 142 further impedes back-flow, as indicated by the diminishing sized arrows labeled "BF" (back-flow). In further aspects, the insufflation fluid entering the shaft also may encourage the fluid to flow into the convection channels, further turning the back-flow into itself via the check valve 120.

It is contemplated that the presently disclosed housing 100 and check valve 120 may be used in other surgical instruments such as a vascular access device. In such a configuration, the housing 100 with the check valve is coupled to a flexible tube with a needle that is insertable into a patient. The vascular access device allows for insertion and removal of vascular access instruments while minimizing blood loss.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

The invention may be described by reference to the following numbered paragraphs:-
1. A surgical access device comprising:
   a tube;
   a housing having a first housing half and a second housing half;
   baffling discs configured to form a lumen, each baffling disc is a conical annulus, and each baffling disc is stacked adjacent to another baffling disc and spaced apart via spacers disposed on each baffling disc so as to define a first gap between adjacent baffling discs for defining a first fluid path; and
   a cover coupled to a proximal end of the housing and configured to control access to the lumen;
   wherein the first and second housing halves of the housing are configured to receive the tube and the baffling discs, the baffling discs spaced apart from the first and second housing halves defining a second gap therebetween for defining a second fluid path;
   wherein the first and second gaps form fluid convection channels for guiding fluid flowing in a proximal direction.
2. The surgical access device of paragraph 1, wherein the first and second housing halves include recesses configured to follow an upper guide of each baffling disc so as to further define the second gap therebetween;
   whereby, when the baffling discs are received by the first and second housing halves, fluid flowing in the proximal direction is encouraged to flow through the fluid convection channels, through the first gap between each baffling disc, through the second gap between the baffling discs and the recesses, and back around against the fluid flowing in the proximal direction so as to reduce net flow in the proximal direction.
3. The surgical access device of paragraph 1, wherein each baffling disc has a cross-sectional profile of an airfoil, with an upper guide and a lower guide, wherein the upper guide has a radius that is larger than a radius of the lower guide.
4. The surgical access device of paragraph 1, wherein the lumen formed by the baffling discs is configured to receive a laparoscopic instrument.
5. The surgical access device of paragraph 1, further including an accessory attachment configured to couple to the cover.
6. The surgical access device of paragraph 5, wherein the accessory attachment includes at least one luer fitting.
7. The surgical access device of paragraph 1, wherein the housing includes five baffling discs.
8. The surgical access device of paragraph 1, wherein the baffling discs define a lumen that is as long as at least a quarter of a length of the tube.
9. The surgical access device of paragraph 1, wherein the tube has a flared proximal end and the first and second housing halves have distal portions configured to couple to and engage the flared proximal end.
10. The surgical access device of paragraph 1, wherein the lumen formed by the baffling discs has a diameter equal to an inner diameter of the tube and is concentric and axially aligned with the tube.
11. A surgical assembly comprising:
   a surgical instrument having a shaft; and
   a surgical access assembly including:
      a housing having open proximal and distal ends,
      a tube extending from the housing,
      baffling discs disposed within the housing, each baffling disc having a cross-sectional profile of an airfoil, each baffling disc spaced apart from an adjacent baffling disc thereby defining a first gap therebetween, each baffling disc spaced from an inner surface of the housing thereby defining a second gap therebetween, the first and second gaps form fluid convection channels for guiding fluid flowing in the housing.
12. The surgical assembly of paragraph 11, wherein the housing includes first and second housing halves.
13. The surgical assembly of paragraph 12, wherein each housing half includes recesses configured to follow an upper guide of each baffling disc so as to further define the second gap therebetween and fluid flowing in a proximal direction is encouraged to flow through the fluid convection channels, through the first gap between each baffling disc, through the second gap between the baffling discs and the recesses, and back around against the fluid flowing in the proximal direction so as to reduce net flow in the proximal direction.
14. The surgical assembly of paragraph 11, wherein the cross-sectional profile of an airfoil is defined by an upper guide and a lower guide, wherein the upper guide has a radius that is larger than a radius of the lower guide.
15. The surgical assembly of paragraph 12, wherein the tube has a flared proximal end and the first and second housing halves have distal portions configured to couple to and engage the flared proximal end.
16. The surgical assembly of paragraph 11, wherein each baffling disc is a conical annulus.
17. The surgical assembly of paragraph 16, wherein a narrow portion of the conical annulus of each baffling disc is positioned distally relative to a user, and is positioned adjacent a wider portion of the conical annulus of an adjacent baffling disc.
18. The surgical assembly of paragraph 17, wherein a proximalmost portion of the tube is spaced apart from the distalmost baffling disc and is configured to follow the narrow portion of the conical annulus of the distalmost baffling disc so as to define a third gap therebetween.
19. The surgical assembly of paragraph 11, further including a cover disposed on a proximalmost portion of the housing of the surgical access assembly.
20. The surgical assembly of paragraph 19, wherein the cover is spaced apart from the proximalmost baffling disc and is configured to follow a portion of the proximalmost baffling disc so as to define a third gap therebetween that forms an extension of the proximalmost fluid convection channel.

## Claims

1. A surgical access device comprising:
a tube;
a housing having a first housing half and a second housing half;
baffling discs configured to form a lumen, each baffling disc is a conical annulus, and each baffling disc is stacked adjacent to another baffling disc and spaced apart via spacers disposed on each baffling disc so as to define a first gap between adjacent baffling discs for defining a first fluid path; and
a cover coupled to a proximal end of the housing and configured to control access to the lumen;
wherein the first and second housing halves of the housing are configured to receive the tube and the baffling discs, the baffling discs spaced apart from the first and second housing halves defining a second gap therebetween for defining a second fluid path;
wherein the first and second gaps form fluid convection channels for guiding fluid flowing in a proximal direction.

2. The surgical access device of claim 1, wherein the first and second housing halves include recesses configured to follow an upper guide of each baffling disc so as to further define the second gap therebetween;
whereby, when the baffling discs are received by the first and second housing halves, fluid flowing in the proximal direction is encouraged to flow through the fluid convection channels, through the first gap between each baffling disc, through the second gap between the baffling discs and the recesses, and back around against the fluid flowing in the proximal direction so as to reduce net flow in the proximal direction.

3. The surgical access device of claim 1 or claim 2, wherein each baffling disc has a cross-sectional profile of an airfoil, with an upper guide and a lower guide, wherein the upper guide has a radius that is larger than a radius of the lower guide.

4. The surgical access device of any preceding claim, wherein the lumen formed by the baffling discs is configured to receive a laparoscopic instrument; and/or further including an accessory attachment configured to couple to the cover; preferably wherein the accessory attachment includes at least one luer fitting.

5. The surgical access device of any preceding claim, wherein the housing includes five baffling discs; and/or wherein the baffling discs define a lumen that is as long as at least a quarter of a length of the tube.

6. The surgical access device of any preceding claim, wherein the tube has a flared proximal end and the first and second housing halves have distal portions configured to couple to and engage the flared proximal end.

7. The surgical access device of any preceding claim, wherein the lumen formed by the baffling discs has a diameter equal to an inner diameter of the tube and is concentric and axially aligned with the tube.

8. A surgical assembly comprising:
a surgical instrument having a shaft; and
a surgical access assembly including:
a housing having open proximal and distal ends,
a tube extending from the housing,
baffling discs disposed within the housing, each baffling disc having a cross-sectional profile of an airfoil, each baffling disc spaced apart from an adjacent baffling disc thereby defining a first gap therebetween, each baffling disc spaced from an inner surface of the housing thereby defining a second gap therebetween, the first and second gaps form fluid convection channels for guiding fluid flowing in the housing.

9. The surgical assembly of claim 8, wherein the housing includes first and second housing halves.

10. The surgical assembly of claim 9, wherein each housing half includes recesses configured to follow an upper guide of each baffling disc so as to further define the second gap therebetween and fluid flowing in a proximal direction is encouraged to flow through the fluid convection channels, through the first gap between each baffling disc, through the second gap between the baffling discs and the recesses, and back around against the fluid flowing in the proximal direction so as to reduce net flow in the proximal direction.

11. The surgical assembly of any of claims 8 to 10, wherein the cross-sectional profile of an airfoil is defined by an upper guide and a lower guide, wherein the upper guide has a radius that is larger than a radius of the lower guide.

12. The surgical assembly of any of claims 8 to 11, wherein the tube has a flared proximal end and the first and second housing halves have distal portions configured to couple to and engage the flared proximal end.

13. The surgical assembly of any of claims 8 to 12, wherein each baffling disc is a conical annulus; preferably wherein a narrow portion of the conical annulus of each baffling disc is positioned distally relative to a user, and is positioned adjacent a wider portion of the conical annulus of an adjacent baffling disc.

14. The surgical assembly of claim 13, wherein a proximalmost portion of the tube is spaced apart from the distalmost baffling disc and is configured to follow the narrow portion of the conical annulus of the distalmost baffling disc so as to define a third gap therebetween.

15. The surgical assembly of any of claims 8 to 14, further including a cover disposed on a proximalmost portion of the housing of the surgical access assembly; preferably wherein the cover is spaced apart from the proximalmost baffling disc and is configured to follow a portion of the proximalmost baffling disc so as to define a third gap therebetween that forms an extension of the proximalmost fluid convection channel.
